# EUROPEAN PATENT SPECIFICATION

(11) **EP 0 558 145 B1**
(45) Date of publication and mention of the grant of the patent: **26.06.1996**
(21) Application number: 93200516.8
(22) Date of filing: 24.02.1993
(51) Int. Cl.: G03C 7/38, C07D 487/04

(54) **Photographic material and process comprising a pyrazolotriazole coupler**
Photographisches Material und Verfahren, das Pyrazolotriazolkuppler enthält
Matériau photographique et procédé comprenant un coupleur pyrazolotriazole

(30) Priority: 26.02.1992 US 841575
(43) Date of publication of application: 01.09.1993
(73) Proprietor: EASTMAN KODAK COMPANY, Rochester, New York 14650-2201 (US)
(72) Inventor: Tang, Ping Wah, c/o EASTMAN KODAK COMPANY, Rochester, New York 14650-2201 (US); Krishnamarthy, Sundaram, c/o EASTMAN KODAK COMPANY, Rochester, New York 14650-2201 (US); Cowan, Stanley, c/o EASTMAN KODAK COMPANY, Rochester, New York 14650-2201 (US)
(74) Representative: Baron, Paul Alexander Clifford

(56) References cited:
- EP-A- 0 178 166
- EP-A- 0 401 610
- EP-A- 0 416 481
- US-A- 4 540 654

## Description

This invention relates to novel pyrazolotriazole dye-forming couplers and to photographic silver halide materials and processes using such couplers.

Color images are customarily obtained in the photographic art by reaction between an oxidation product of an silver halide developing agent and a dye-forming coupler. Pyrazolone couplers are useful for forming magenta dye images; however, pyrazoloazole couplers, particularly pyrazolotriazole couplers, represent another class of couplers for this purpose. Examples of pyrazolotriazole couplers are described in, for example, U.S. Patent 4,443,536; U.S. Patent Nos. 1,247,493; 1,252,418; and 1,398,979; and U.S. Patents 4,665,015; 4,514,490; 4,540,654; 4,590,153; 4,822,730; 5,132,202 and European Patent 177765. One class of pyrazolotriazole couplers includes 1H-pyrazolo[1,5-b]1,2,4 triazole couplers, such as described in European Patent 177765.

While such magenta dye-forming couplers are useful in photographic silver halide materials and processes, many of the dyes formed from such couplers do not have sufficient light stability. It has been desirable to provide a lH-pyrazolo[1,5-b]1,2,4 triazole coupler enabling formation of a dye that exhibits increased light stability, said coupler comprising in the 6- position an unsubstituted or substituted adamantyl group.

Such dye-forming couplers are particularly useful in photographic silver halide materials and processes, especially photographic silver halide color paper materials and processes.

It is believed that the adamantyl group in the 6- position of the described coupler enables formation of a dye exhibiting increased stability due to the particular nature of the combination of the adamantyl group and the particular 1H-pyrazolo[1,5-b]1,2,4 triazole nucleus. The unique rigidness of the multicyclic structure of the adamantyl group within the molecule is believed to play a role in enabling the increased light stability by reducing the ability of the nucleus to decompose when exposed to light. For example, an adamantyl group is unique by virtue of its diamond-like structure and its ability to provide a fully substituted carbon center without creating excessive steric hindrance. A conventional t-butyl group provides more steric hindrance that is undesired.

According to the present invention there is provided a photographic element comprising a support bearing at least one photographic silver halide emulsion layer and a dye-forming lH-pyrazolo [1,5-b]1,2,4 triazole coupler of the formula: 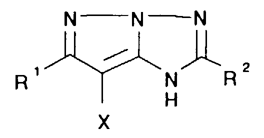 wherein
R¹ is a substituted or unsubstituted adamantyl group;
R² is a substituent group; and
X is hydrogen or a coupling-off group.

The term A1 herein means an adamantyl group. Illustrative examples of useful couplers as described are as follows:

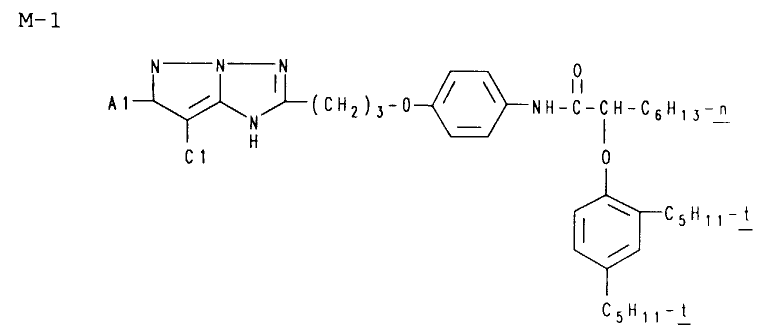

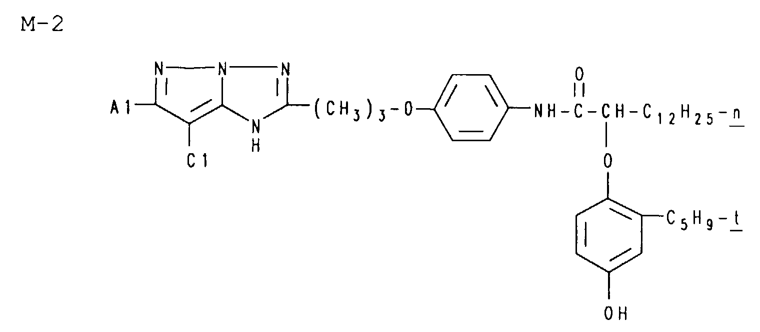

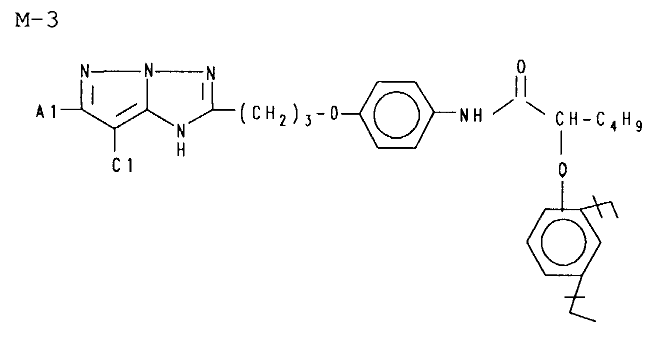

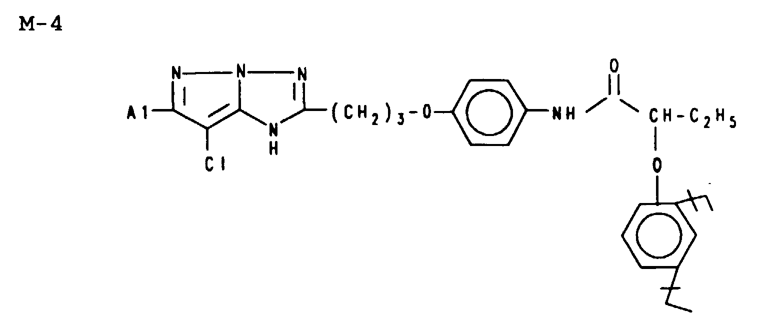

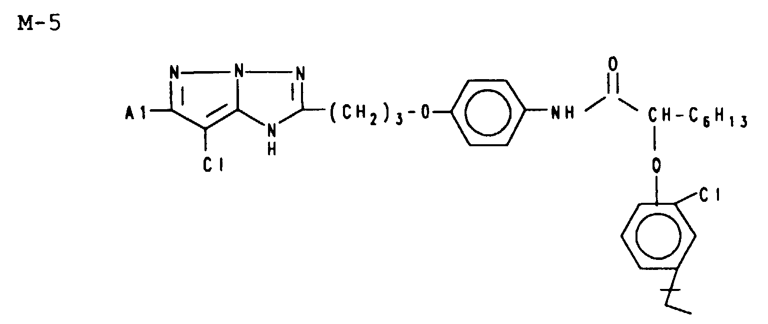

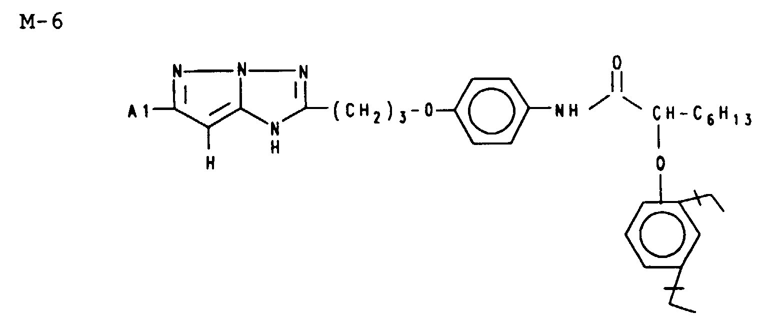

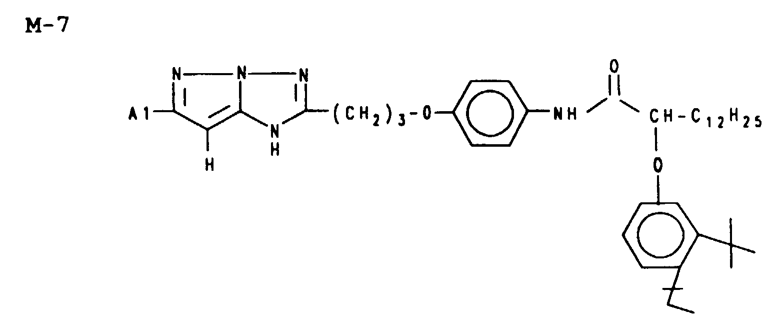

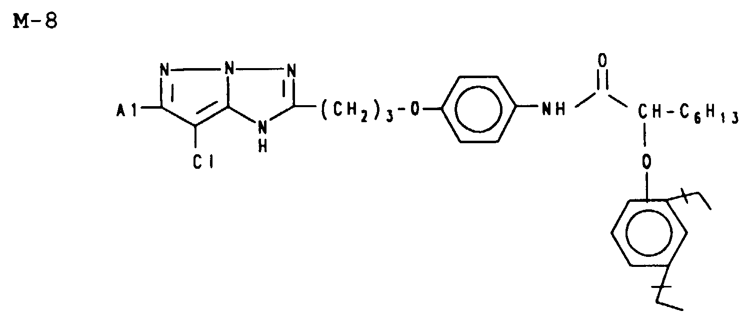

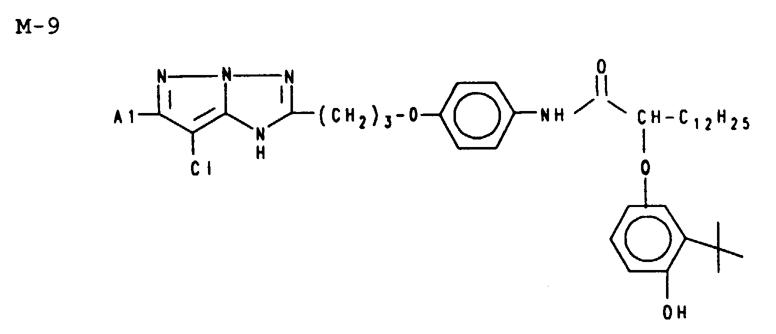

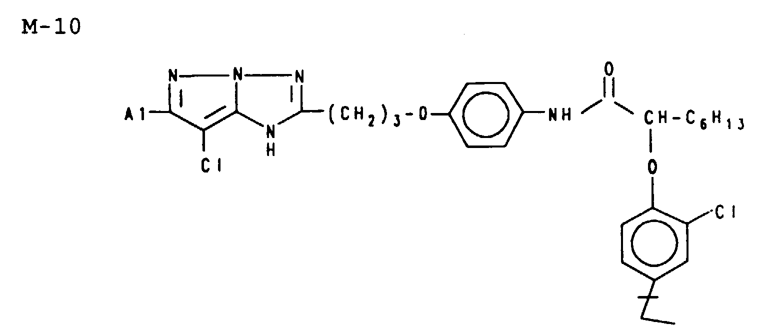

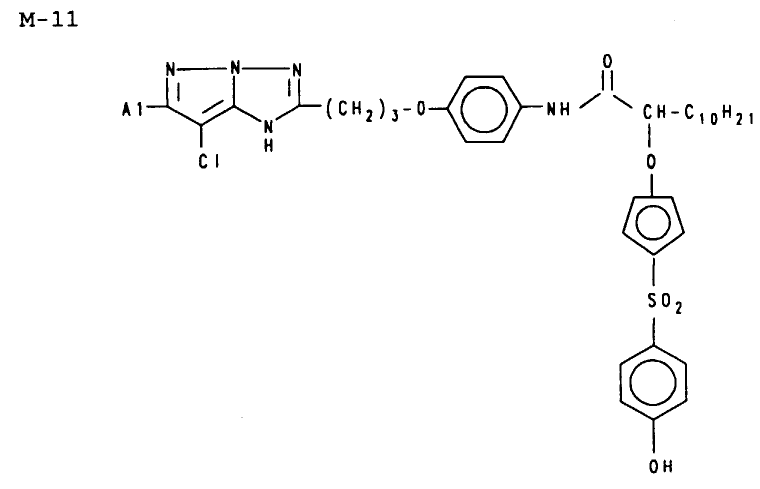

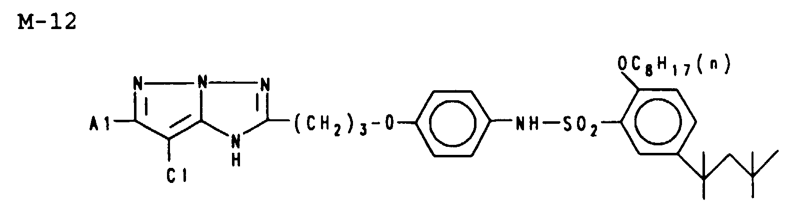

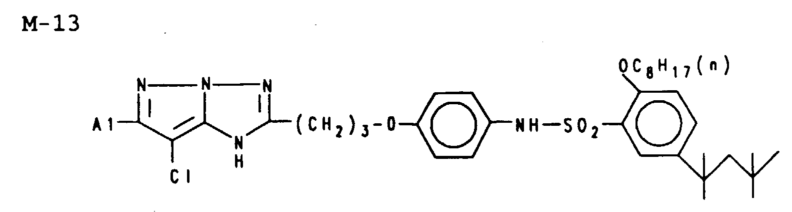

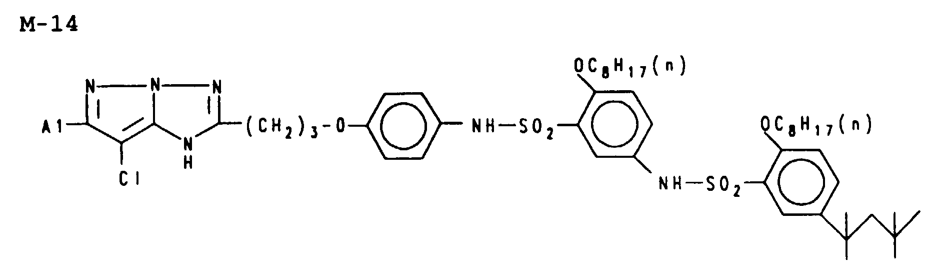

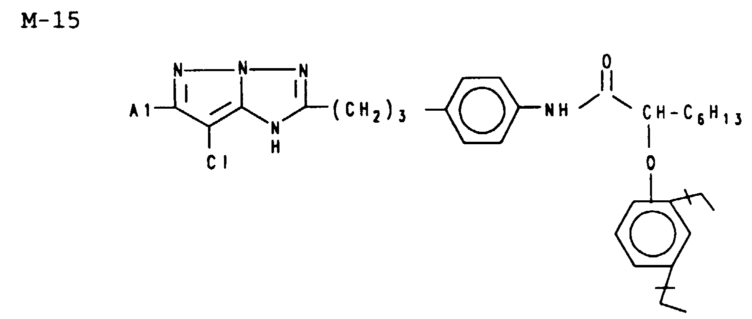

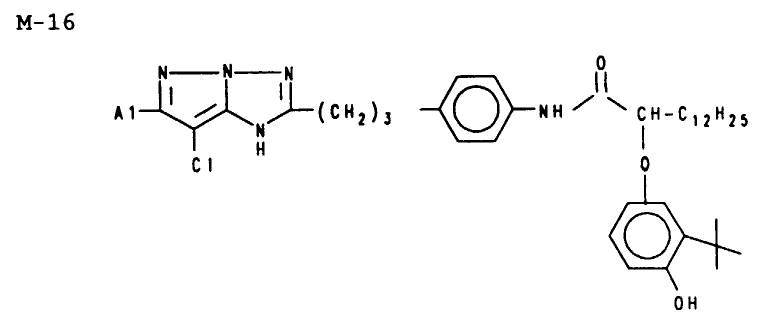

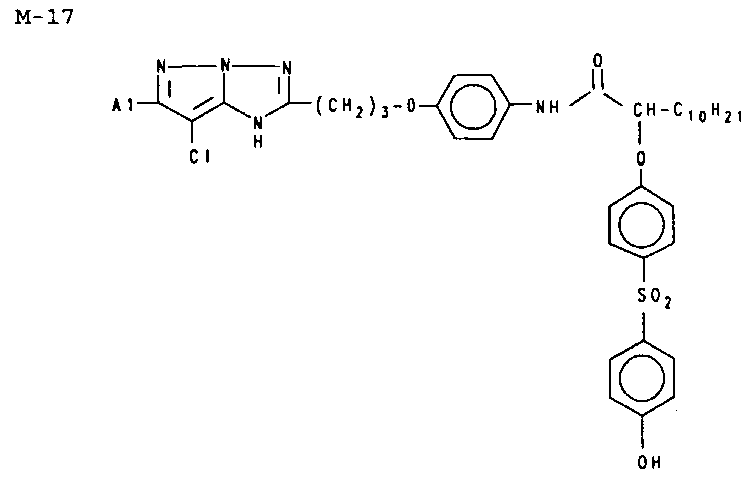

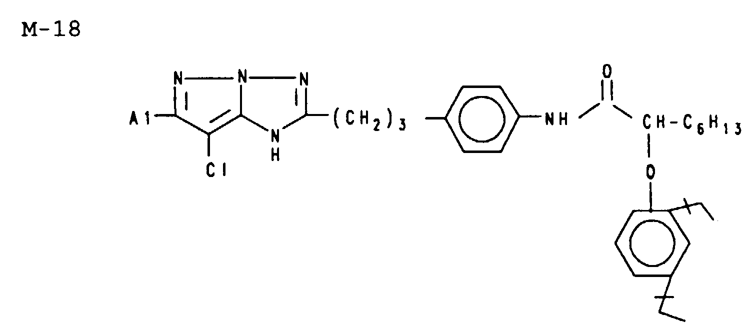

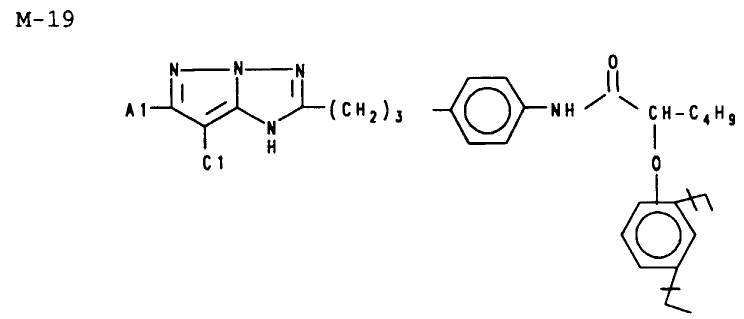

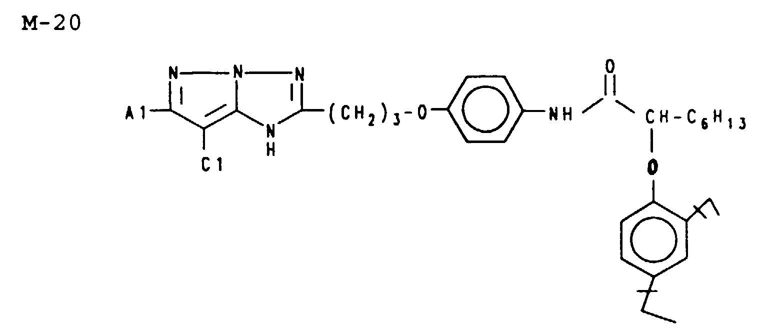

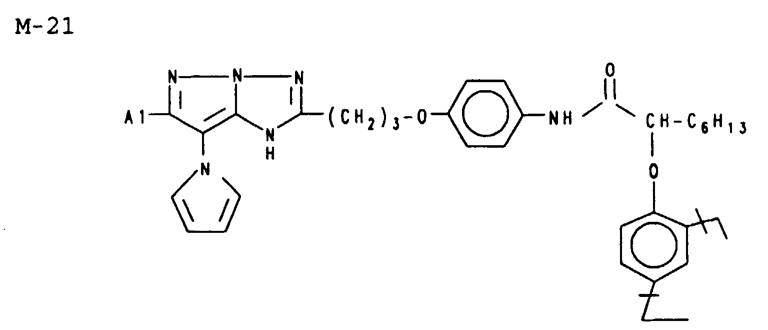

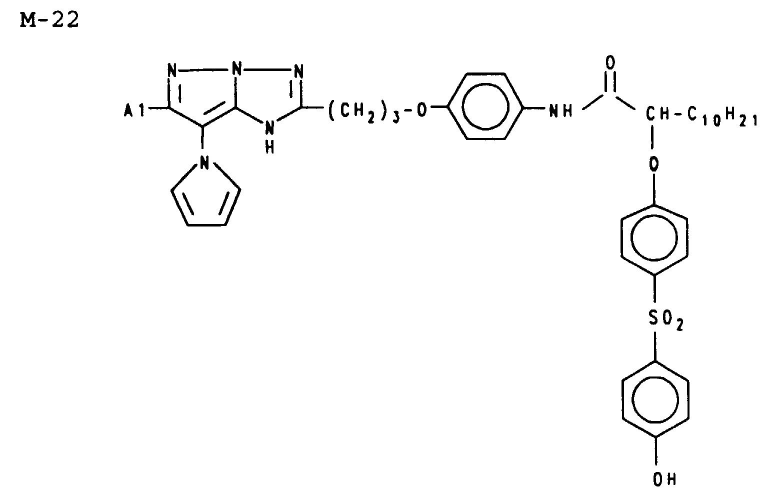

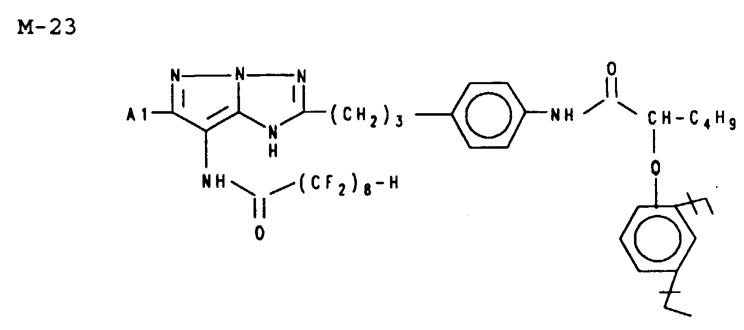

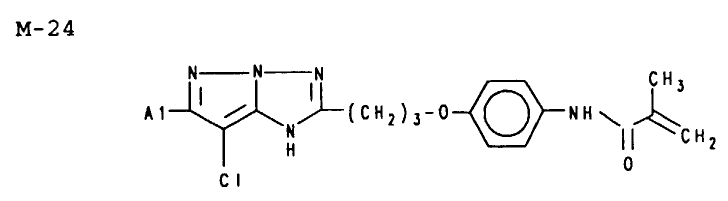

The pyrazolotriazole coupler typically contains, in a position that does not contain the described adamantyl group, hydrogen or group that typically promotes solubility, diffusion resistance or dye hue of the dye formed form upon reaction of the coupler with the oxidized color developing agent.

The pyrazolotriazole coupler typically contains, in a position not containing the adamantyl group as described, hydrogen or a group selected from the following: amino, such as dioctylamino, dimethylamino, and dodecylamino; alkyl, such as straight or branched chain alkyl containing 1 to 40 carbon atoms, for example methyl, ethyl, n-propyl, n-butyl, t-butyl, and dodecyl; cycloalkyl, such as s cyclohexyl and cyclopentyl; aryl, such as s aryl containing 6 to 30 carbon atoms, for example, phenyl, naphthyl and mesityl; carboxy; cyano; nitro; a heterocyclic group, such as heterocyclic group comprised of atoms selected from carbon, oxygen, nitrogen, and sulfur atoms necessary to complete a five or six member ring, for example furyl, pyrrolyl, oxazolyl, thienyl, thiazolyl, and pyridyl; and groups as described in U.S. Patent 4,948,722, the disclosure of which is incorporated herein by reference.

These groups are unsubstituted or substituted with groups that do not adversely affect the desired properties of the pyrazoloazole coupler. Examples of useful substituents include ballast groups and coupler moieties that are known to be useful in the photographic art, or alkyl, such as alkyl containing 1 to 4 carbon atoms, for example, methyl, ethyl, and t-butyl.

The pyrazolotriazole contains in the coupling position, hydrogen or a coupling-off group, also known as a leaving group.

Coupling-off groups are known to those skilled in the art. Such groups can determine the equivalency of the coupler, can modify the reactivity of the coupler, or can advantageously affect the layer in which the coupler is coated or other layers in the element by performing, after release from the coupler, such functions as development inhibition, development acceleration, bleach inhibition, bleach acceleration, color correction, and the like. Representative classes of coupling-off groups include halogen, particularly chlorine, bromine, or fluorine, alkoxy, aryloxy, heterocyclyloxy, heterocyclic, such as hydantoin and sulfonyloxy, pyrazolo groups, acyloxy, carbonamido, imido, acyl, heterocyclylimido, thiocyano, alkylthio, arylthio, heterocyclylthio, sulfonamido, phosphonyloxy and arylazo. They are described in, for example, U.S. Patents 2,355,169; 3,227,551; 3,432,521; 3,476,563; 3,617,291; 3,880,661; 4,052,212 and 4,134,766; and in U.K. patents and published application numbers 1,466,728; 1,531,927; 1,533,039; 2,006,755A and 2,017,704A; the disclosures of which are incorporated herein by reference.

Examples of specific coupling-off groups are 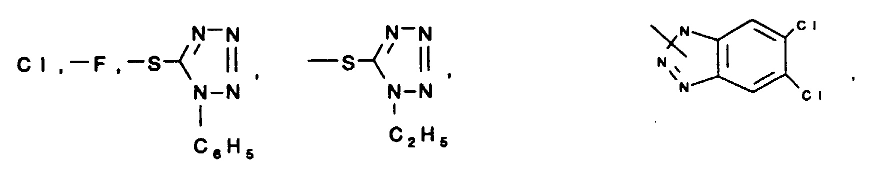 -SCN, -OCH₃ OC₆H₅, -OCH₂CONHCH₂CH₂OH, -OCH₂CONHCH₂CH₂OCH₃, -OCH₂CONHCH₂CH₂OCOCH₃,-NHSO₂CH₃

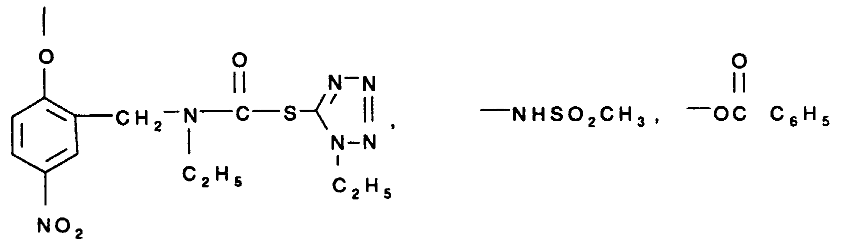

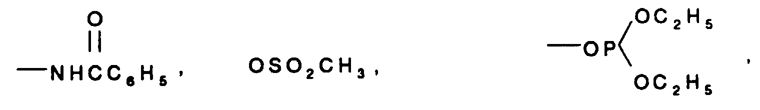

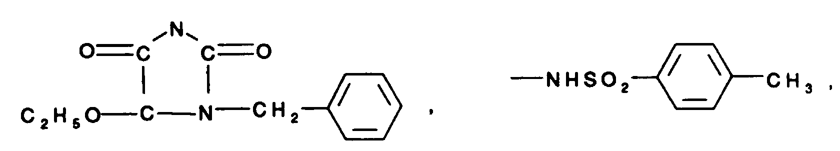

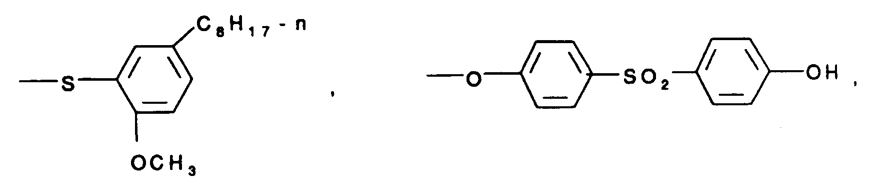

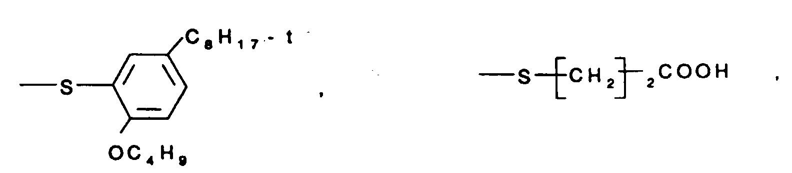

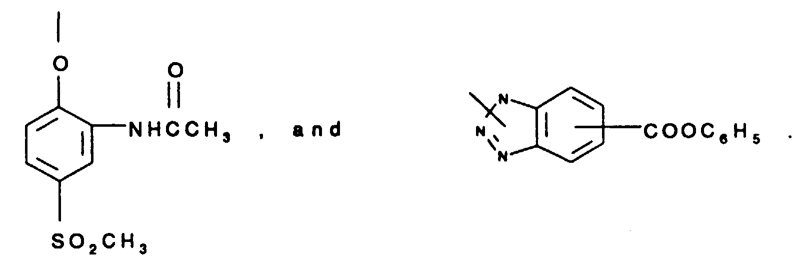

The pyrazolotriazoles typically comprise a ballast group. A ballast group as described is an organic radical of such size and configuration as to confer on the coupler molecule sufficient bulk to render the coupler substantially non-diffusible from the layer in which it is coated in a photographic element. Couplers of the invention may be attached to ballast groups, or to polymeric chains through one or more of the groups on the pyrazolotriazole nucleus. For example, one or more coupler moieties can be attached to the same ballast group. Representative ballast groups include substituted or unsubstituted alkyl or aryl groups containing 8 to 32 carbon atoms. Representative substituents include alkyl, aryl, alkoxy, aryloxy, alkylthio, arylthio, hydroxy, halogen, alkoxycarbonyl, aryloxycarbonyl, carboxy, acyl, acyloxy, carbonamido, carbamoyl, alkylsulfonyl, arylsulfonyl, sulfonamido, and sulfamoyl groups wherein the alkyl and aryl substituents and the alkyl and aryl portions of the alkoxy, aryloxy, alkylthio, arylthio, alkoxycarbonyl, arylcarbonyl, acyl, acyloxy, carbonamido, carbamoyl, alkylsulfonyl, arylsulfonyl, sulfonamido, and sulfamoyl substituents containing 1 to 30 carbon atoms and 6 to 30 carbon atoms, respectively, can be further substituted with such substituents.

Pyrazolotriazole couplers as described can be used in ways and for purposes that pyrazolotriazole couplers have been used in the photographic art.

Pyrazolotriazole couplers as described are prepared by general methods of synthesis described in the art, such as in U.S. Patent No. 4,540,654. An illustrative synthesis Scheme I is as follows:

### Scheme I

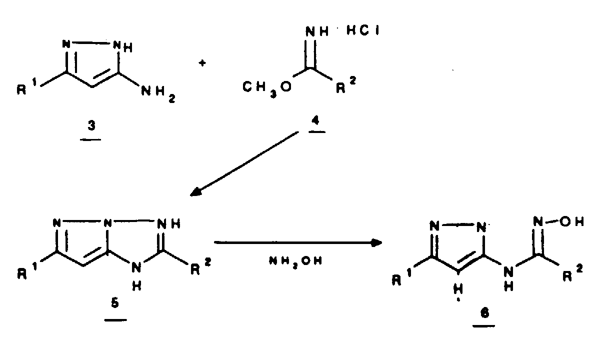

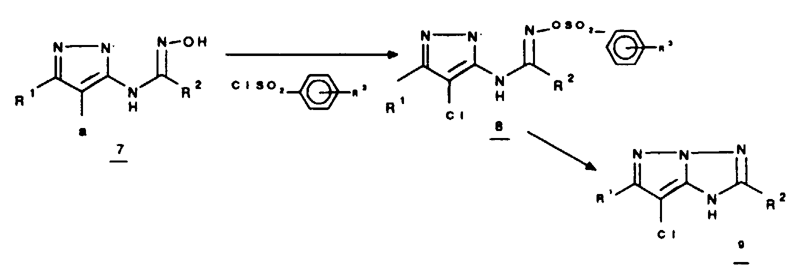

wherein R¹ and R² are as described; and, R³ is hydrogen or a substituted that does not adversely affect the reaction, such as alkyl containing 1 to 3 carbon atoms.

### Synthesis Example A:

An example of synthesis of a coupler as described is as follows:

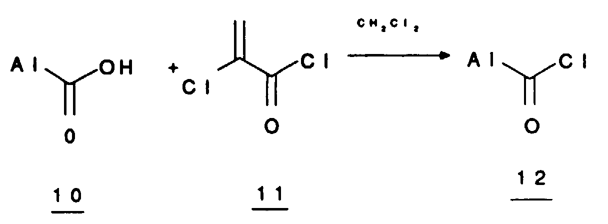

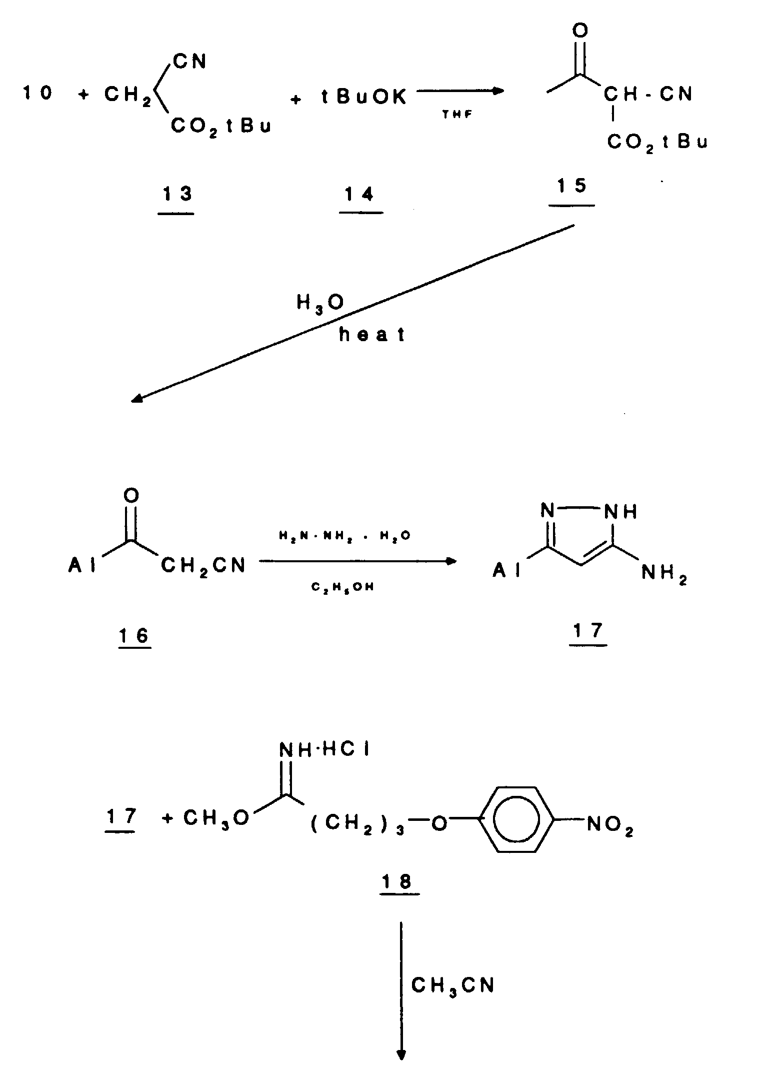

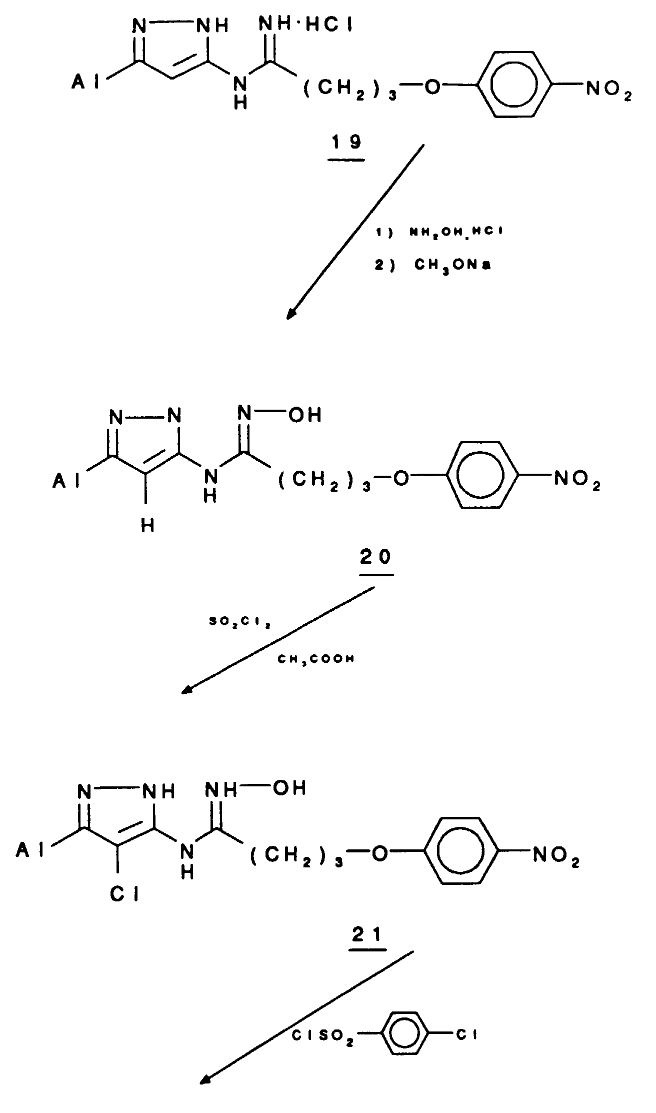

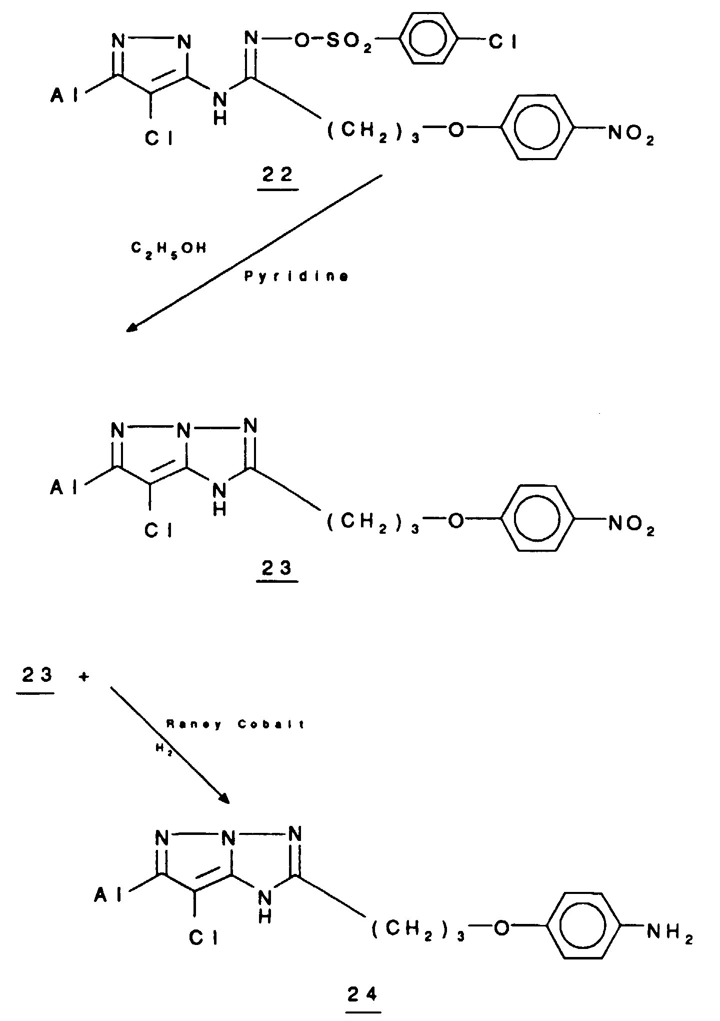

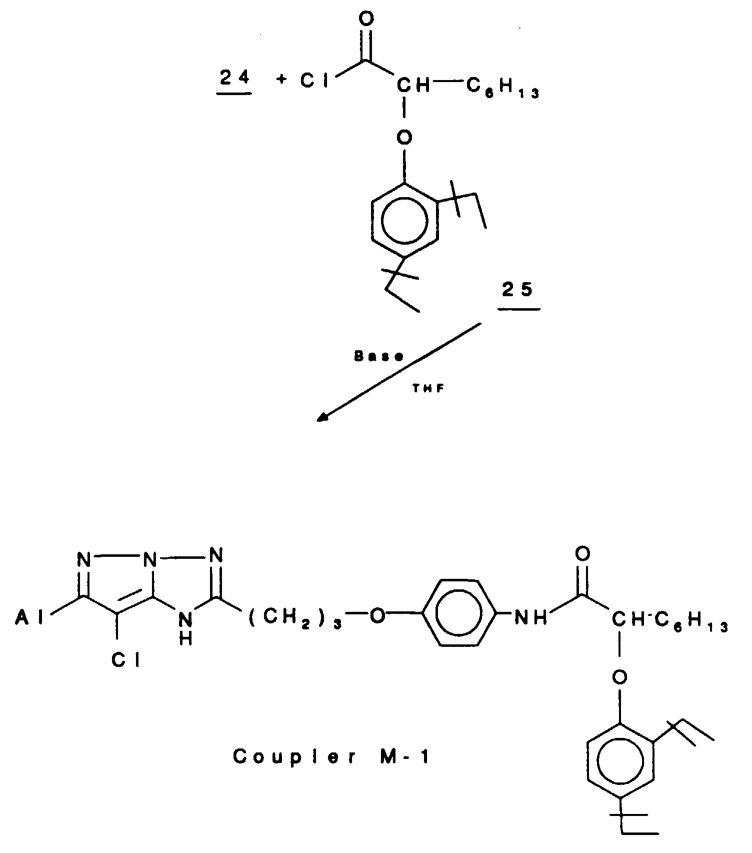

### Preparation of 1-Adamantyl cyanomethyl ketone (16)

In a one liter, round-bottomed flask equipped with a mechanical stirrer 14.12 g (0.10 mol) of t-butyl cyanoacetate was dissolved in 300 mL of dried THF. 12.35 g (0.11 mol) of potassium t-butoxide was added over a period of 15 minutes by means of a solid addition funnel. The reaction was stirred at 25°C for 20 minutes, followed by the drop wise addition of 0.10 mole of adamantoyl chloride (12) (in 20 mL of THF), prepared by reaction of 1-adamanecarboxylic acid (10) and oxalyl chloride (11). After the addition had been completed, the reaction was heated at reflux under N₂ for 18 hours. To the cooled stirred reaction mixture was added 23.8 g (0.125 mol) of p-toluenesulfonic acid monohydrate dissolved in 70 mL of water. The reaction was heated at reflux for 18 hours and then cooled to 20°C. The mixture was saturated with sodium chloride and diluted with ether. The layers were partitioned and the aqueous layer was extracted twice with ether. The combined organic extracts were washed with a saturated brine solution, dried and concentrated in vacuo to yield a pale yellow solid which was triturated with 200 mL of a saturated solution of potassium bicarbonate for 18 hours. The solid was collected, washed with water and dried. The yield of the desired product (16) was 10.15 g (50%). All the analytical data were in agreement with the structure.

### Preparation of 5-amino-3-adamantyl pyrazole (17)

To a suspension of 20.33g (0.10 mol) of 1-adamantyl cyanomethyl ketone (16) in 100 mL of ethanol at 20°C was added 7.25 g (0.145 mol) of hydrazine monohydrate. The mixture was heated at reflux for 18 hours and then cooled to 25°C. The cloudy solution was filtered and the filtrate was concentrated in vacuo to yield a pale yellow oil. The oil was dissolved in 60 mL of dichloromethane, and the solution was concentrated in vacuo to afford a yellow oil. The remaining solvent was removed by vacuum evaporation (0.5 mm Hg) to yield 21.9 g (100%) of pyrazole (17) as a thick yellow oil which solidified to a waxy solid upon standing at 25°C. The product was fully characterized by IR and 'H NMR analyses, and all the data were in agreement with the assigned structure.

### Preparation of oxime (20)

To a solution of 25 g (0.115 mol) of pyrazole (17) in 250 mL of dried acetonitrile was added 32.9 g (0.12 mol) of imidate (18) over a period of 10 minutes by means of a solid addition funnel. The mixture was vigorously stirred at 25°C for 18 hours and was concentrated in vacuo to afford an off-white solid. To the well-stirred solid were added 100 mL of dried methanol and 8.33 g (0.12 mol) of hydroxylamine hydrochloride. The suspension was stirred for several minutes, followed by the addition of 240 mL of a 0.5 M solution of sodium methoxide in methanol (0.12 mol) over a period of 30 minutes by means of an addition funnel. The reaction was stirred at 25°C for 18 hours and then poured into a mixture of ice-water. The mixture was stirred for 30 minutes. The resulting solid was collected, washed well with cold methanol and dried. The yield of the desired oxime (20) was 32.85 g (65%). The product was fully characterized by 'HNMR and combustion analysis. All the data were consistent with the assigned structure.

### Preparation of oxime (21)

A solution of 2.20 g (5m mol) of oxime (20) dissolved in 14 mL of glacial acetic acid was cooled to 15°C in an ice-water bath, followed by the drop wise addition of 0.782 g (0.58 mol) of sulfuryl chloride. After the addition had been completed, the cold bath was removed, the reaction was allowed to warm up to 25°C and was stirred for an additional 2 hours. The reaction was poured into a mixture of ice-water containing 1.0 g (12m mol) of sodium bicarbonate. the mixture was stirred for 30 minutes and the solid was collected, washed thoroughly with water, and dried. the yield of the desired chloro-compound (21) was 2.24 g (94.5%). The product was fully characterized by 'HNMR and combustion analysis. All the data were consistent with the assigned structure.

### Preparation of the Benzene Sulfonate Intermediate (22)

A mixture of 273.4 g (0.58 mol) of oxime (21) in 4 liters of dried THF was cooled at 0°C, followed by the addition of 50.0 g (0.63 mol) of pyridine. 131.10 g (0.621 mol) of 4-chlorobenzene sulfonyl chloride was added over a period of 15 minutes by means of a solid addition funnel. After the addition had been completed, the reaction was stirred at 0°C for another 30 minutes. The cold bath was removed and the reaction was stirred at ambient temperature for 1.5 hours. The reaction was filtered and the filtrate was poured into a mixture of ice-water containing 80 mL of concentrated hydrochloric acid and extracted three times with ethyl acetate. The combined organic extracts were washed with a saturated brine solution, dried over anhydrous magnesium sulfate and filtered. The filtrate was concentrated in vacuo to afford 348.70 g (92.7%) of a tan solid. Physical and spectroscopic data of the product were consistent with the assigned structure.

### Preparation of Coupler (23)

To a mixture of 348.70 g (0.537 mol) of benzenesulfonate (22) in 4 liter of ethanol was added portion wise 42.40 g (0.537 mol) of pyridine. The reaction was heated at 60°C for 1 hour under an inert atmosphere. The mixture was poured into a mixture of ice-water containing 70 mL of concentrated hydrochloric acid and stirred for 1 hour. The resulting solid was collected, washed successively with water and with ligroin. The crude product was triturated in dichloromethane. The mixture was filtered under suction to afford 125.80 g (52%) of the desired coupler (23). The product was fully characterized by 'HNMR HPLC and combustion analysis. All the data were in agreement with the assigned structure.

### Preparation of the Coupler amine (24)

The nitro-compound (23) (125.8 g, 0.276 mol) dissolved in THF was reduced to the corresponding amine (24) using Raney Cobalt as catalyst. The reaction mixture was filtered and the filtrate was concentrated to afford 84.60 g (72%) of coupler amine (24) as a tan solid. Physical and spectroscopic data of the amine were consistent with the assigned structure.

### Preparation of 7-chloro-6-adamantyl-2-(3-(4-(2-(2,4-bis(1,1,dimethyl propyl) phenoxy)-1H-pyrazolo[1,5-b]1,2,4 triazole: Coupler

In a 250-mL three-necked round-bottomed flank equipped with a magnetic stir bar and an addition funnel 3.10 g (7.27m mol) of coupler-amine (24) and 1.02 g (8.40m mol) of N,N-dimethylamiline were added to 30 mL of dried THF. To the ice-cooled stirred reaction mixture was added drop wise a solution of 3.302 g (8.36m mol) of 2-(2,4-bis(1,1-dimethyl propyl)phenoxy)octanoyl chloride dissolved in 20 mL of THF. After the addition had been completed, the mixture was stirred at 2°C under an inert atmosphere for an additional 2 hours and then poured into a mixture of ice-water containing 2 mL of concentrated hydrochloric acid. After the mixture had been stirred for 2 hours, the resulting solid was collected, washed with water and dried. The product was essentially pure as observed by thin layer chromatography. Flash chromatography (silica gel, 30% AcOEt/Ligrom) yielded 4.56 g (80%) of 1 as a white solid. The product was fully characterized by 'HNMR and HPLC. The data were in agreement with the assigned structure. Anal. Caled for C₄₇H₆₆ClN₅O₃: C, 71.95; H, 8.48; N, 8.92; Cl, 4.51. Found: C, 71.84; H, 8.41; N, 8.89; Cl, 4.47.

The couplers of this invention can be incorporated in silver halide emulsions and the emulsions can be coated on a support to form a photographic element. Alternatively, the couplers can be incorporated in photographic elements adjacent the silver halide emulsion where, during development, the coupler will be in reactive association with development products such as oxidized color developing agent.

The photographic elements can be either single color or multicolor elements. In a multicolor element, the magenta dye-forming coupler is usually associated with a green-sensitive emulsion, although they could be associated with an unsensitized emulsion or an emulsion sensitized to a different region of the spectrum. Multicolor elements contain dye image-forming units sensitive to each of the three primary regions of the spectrum. Each unit can be comprised of a single emulsion layer or of multiple emulsion layers sensitive to a given region of the spectrum. the layers of the element, including the layers of the image-forming units, can be arranged in various orders as known in the art.

A typical multicolor photographic element comprises a support bearing a cyan dye image-forming unit comprising at least one red-sensitive silver halide emulsion layer having associated therewith at least one cyan dye-forming coupler, a magenta image-forming unit comprising at least one green-sensitive silver halide emulsion layer having associated therewith at least one magenta dye-forming coupler and a yellow dye image-forming unit comprising at least one blue-sensitive silver halide emulsion layer having associated therewith at least one yellow dye-forming coupler. the element can contain additional layers, such as filter layers, inter-layers, overcoat layers, subbing layers, and the like.

In the following discussion of suitable materials for use in the elements of this invention, reference will be made to Research Disclosure, December 1978, Item 17643, published by Kenneth Mason Publications, Ltd., The Old Harbourmaster's, 8 North Street, Emsworth, Hampshire PO10 7DD, ENGLAND, and Research Disclosure, Dec. 1989, Item 308119, the disclosures of which are incorporated herein by reference. This latter publication will be identified hereafter by the term "Research Disclosure."

The silver halide emulsions employed in the elements of this invention can be comprised of silver bromide, silver chloride, silver iodide, silver chlorobromide, silver chloroiodide, silver bromiodide, silver chlorobromoiodide or mixtures thereof. The emulsions can include silver halide grains of any conventional shape or size. Specifically, the emulsions can include coarse, medium or fine silver halide grains. High aspect ratio tabular grain emulsions are specifically contemplated, such as those disclosed by Wilgus et al U.S. Patent 4,434,226, Daubendiek et al U.S. Patent 4,414,310, Wey U.S. Patent 4,399,215, Solberg et al U.S. Patent 4,433,048, Mignot U.S. Patent 4,386,156, Evans et al U.S. Patent 4,504,570, Maskasky U.S. Patent 4,400,463, Wey et al U.S. Patent 4,414,306, Maskasky U.S. patents 4,435,501 and 4,643,966 and Daubendiek et al U.S. Patents 4,672,027 and 4,693,964. Also specifically contemplated are those silver bromoiodide grains with a higher molar proportion of iodide in the core of the grain than in the periphery of the grain, such as those described in GB 1,027,146; JA 54/48,521; US 4,379,837; US 4,444,877; US 4,665,012; US 4,686,178; US 4,565,778; US 4,728,602; US 4,668,614; US 4,636,461; EP 264,954. The silver halide emulsions can be either monodisperse or polydisperse as precipitated. The grain size distribution of the emulsions can be controlled by silver halide grain separation techniques or by blending silver halide emulsions of differing grain sizes.

Sensitizing compounds, such as compounds of copper, thallium, lead, bismuth, cadmium and group VIII noble metals, can be present during precipitation of the silver halide emulsion.

The emulsions can be surface-sensitive emulsions, i.e., emulsions that form latent images primarily on the surfaces of the silver halide grains, or internal latent images predominantly in the interior of the silver halide grains. The emulsions can be negative-working emulsions, such as surface-sensitive emulsions or unfogged internal latent image-forming emulsions, or direct-positive emulsions of the unfogged, internal latent image-forming type, which are positive-working when development is conducted with uniform light exposure or in the presence of a nucleating agent.

The silver halide emulsions can be surface sensitized. Noble metal (e.g., gold), middle chalcogen (e.g., sulfur, selenium, or tellurium), and reduction sensitizers, employed individually or in combination, are specifically contemplated. Typical chemical sensitizers are listed in Research Disclosure, Items 17643 and 308119, cited above, Section III.

The silver halide emulsions can be spectrally sensitized with dyes from a variety of classes, including the polymethine dye class, which includes the cyanines, merocyanines, complex cyanines and merocyanines (i.e., tri-, tetra-, and polynuclear cyanines and merocyanines), oxonols, hemioxonols, styryls, merostyryls, and streptocyanines. Illustrative spectral sensitizing dyes are disclosed in Research Disclosure, Items 17643 and 308119, cited above, Section IV.

Suitable vehicles for the emulsion layers and other layers of elements of this invention are described in Research Disclosure Items 17643 and 308119, Section IX and the publications cited therein.

In addition to the couplers described herein the elements of this invention can include additional couplers as described in Research Disclosure, Items 17643 and 308119, Section VII, and the publications cited therein. These additional couplers can be incorporated as described in the above Research Disclosure and the publications cited therein.

The photographic elements of this invention can contain brighteners (Research disclosure Items 17643 and 308119 Section V), antifoggants and stabilizers (Research Disclosure Items 17643 and 308119 Section VI), antistain agents and image dye stabilizers (Research Disclosure Items 17643 and 308119 Section VII, paragraphs I and J), light absorbing and scattering materials (Research Disclosure Items 17643 and 308119 Section VIII), hardeners (Research Disclosure Items 17643 and 308119 Section X), coating aids (Research Disclosure Items 17643 and 308119 section XI), plasticizers and lubricants (Research Disclosure Items 17643 and 308119 Section XII), antistatic agents (Research Disclosure Items 17643 and 308119 Section XIII), matting agents (Research Disclosure Items 17643 and 308119 Section XVI) and development modifiers (Research Disclosure Items 17643 and 308119 Section XXI).

The photographic elements can be coated on a variety of supports as described in Research Disclosure Items 17643 and 308119 Section XVII and the references described therein.

Photographic elements can be exposed to actinic radiation, typically in the visible region of the spectrum, to form a latent image as described in Research disclosure Items 17643 and 308119 Section XVIII and then processed to form a visible dye image as described in research disclosure Items 17643 and 308119 Section XIX. Processing to form a visible dye image includes the step of contacting the element with a color developing agent to reduce developable silver halide and oxidize the color developing agent. Oxidized color developing agent. Oxidized color developing agent in turn reacts with the coupler to yield a dye.

Preferred color developing agents are p-phenylene diamines. especially preferred are 4-amino-3-methyl-N, N-diethylaniline hydrochloride, 4-amino-3-methyl-N-ethyl-N-β-(methanesulfonamido)-ethylaniline sulfate hydrate, 4-amino-3-methyl-N-ethyl-N-β-hydroxyethylaniline sulfate, 4-amino-3-β-(methanesulfonamido)ethyl-N,N-diethylaniline hydrochloride and 4-amino-N-ethyl-N- (2-methoxy-ethyl)-m-toluidine di-p-toluene sulfonic acid.

With negative-working silver halide, the processing step described above provides a negative image. The described elements can be processed in the known C-41 color process as described in, for example, the British Journal of Photography Annual of 1982, pages 209 - 211 and 1988, pages 191-198 or in known processes for processing color photographic papers, such as the known RA-4 of Eastman Kodak Company. The described elements are optionally processed in the known color processes for processing color print papers, such as the processes described in the British Journal of Photography Annual of 1988, pages 198-199. To provide a positive (or reversal) image, the color development step can be preceded by development with a non-chromogenic developing agent to develop exposed silver halide, but not form dye, and then uniformly fogging the element to render unexposed silver halide, but not form dye, and then uniformly fogging the element to render unexposed silver halide developable. Alternatively, a direct positive emulsion can be employed to obtain a positive image.

Development is followed by the conventional steps of bleaching, fixing, or bleach-fixing, to remove silver or silver halide, washing, and drying.

The following examples further illustrate the invention.

Dispersions of the couplers were prepared in the following manner: The quantities of each component are found in Table I. In one vessel, the coupler, stabilizer (2,2',3,3'-tetrahydro-3,3,3',3'-tetramethyl-5,5',6,6'-tetrapropoxy-1,1'-spirobi[1H-indene]), coupler solvent (tritolyl phospate), and ethyl acetate were combined and warmed to dissolve. In a second vessel, gelatin, Alkanol XC (surfactant and Trademark of E. I. Dupont Co., U.S.A.) and water were combined and warmed to about 40°C. The two mixtures were mixed together and passed three times through a Gaulin colloid mill. The ethyl acetate was removed by evaporation and water was added to restore the original weight after milling.

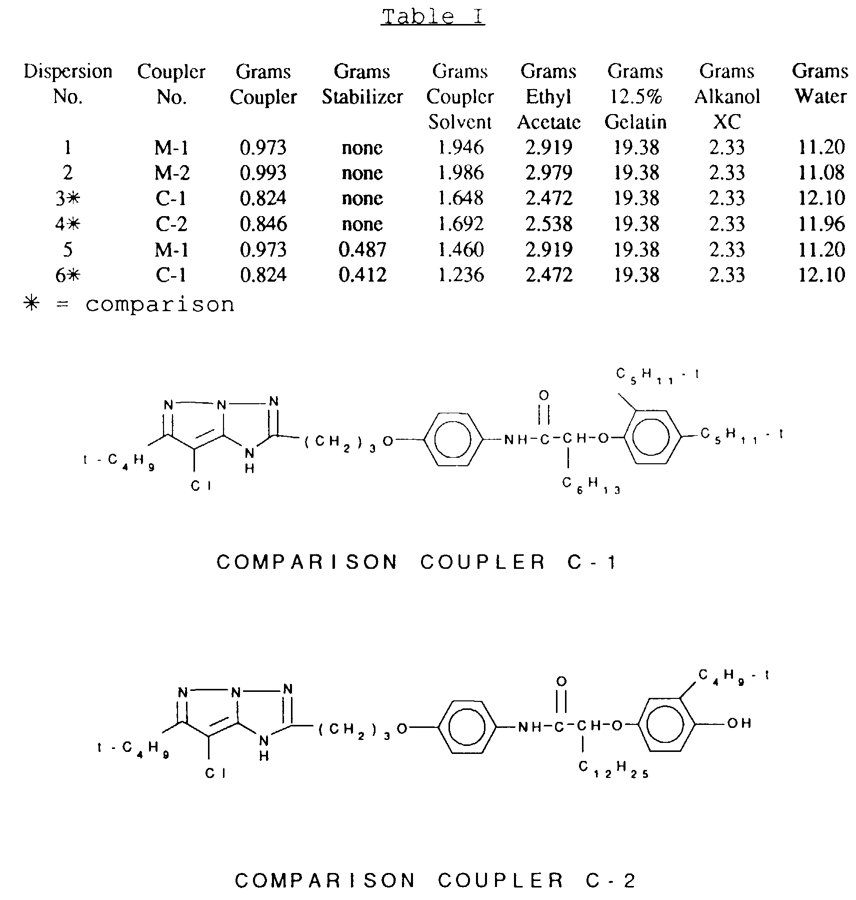

The photographic elements were prepared by coating the following layers in the order listed on a resin-coated paper support:

### 1st Layer

| | |
|---|---|
| Gelatin | 3.23 g/m² |

### 2nd Layer

| | |
|---|---|
| Gelatin | 1.61 g/m² |
| Coupler Dispersion (See TableII) coupler/m² | 4.3 x 10⁻⁷ mole |
| Green-sensitized AgCl gelatin emulsion | 0.17 mg Ag/m² |

### 3rd Layer

| | |
|---|---|
| Gelatin | 1.33 g/m² |
| 2-(2H-benzotriazol-2-yl)-4,6-bis-(1,1-dimethylpropyl)phenol | 0.73 g/m² |
| Tinuvin 326 (U.V. absorber and trademark of Ciba-Grigg Corp., U.S.A) | 0.13 g/m² |

**Table II**

| Example No. | Dispersion No. |
|---|---|
| 1 | 1 |
| 2 | 2 |
| 3* | 3 |
| 4* | 4 |
| 5 | 5 |
| 6* | 6 |

| | |
|---|---|
| * = comparison | |

The photographic elements were given stepwise exposures to green light and processed as follows at 35°C:

| | |
|---|---|
| Developer | 45 seconds |
| Bleach-Fix | 45 seconds |
| Wash (running water) | 1 minute, 30 seconds |

The developer and bleach-fix were of the following compositions:

### Developer

| | |
|---|---|
| Water | 700.00 mL |
| Triethanolamine | 12.41 g |
| Phorwite REU (trademark of ____) | 2.30 g |
| Lithium polystyrene sulfonate (30%) | 0.30 g |
| N,N-Diethylhydroxylamine (85%) | 5.40 g |
| Lithium sulfate | 2.70 g |
| N-{2-[(4-amino-3-methylphenyl)ethylamino]ethyl}-methanesulfonamide, sesquisulfate | 5.00 g |
| 1-Hydroxyethyl-1,1-diphosphonic acid (60%) | 0.81 g |
| Potassium carbonate, anhydrous | 21.16 g |
| Potassium chloride | 1.60 g |
| Potassium bromide | 7.00 mg |
| Water to make | 1.00 L |
| pH @ 26.7°C adjusted to 10.4 ± 0.05 | |

### Bleach-Fix

| | |
|---|---|
| Water | 700.00 mL |
| Solution of ammonium thiosulfate (56.4%) + Ammonium sulfite (4%) | 127.40 g |
| Acetic acid (glacial) | 10.20 g |
| Solution of ammonium ferric ethylenediaminetetraacetate (44%) + ethylenediaminetetraacetic acid (3.5%) | 110.40g |
| Water to make | 1.00 L |
| pH @ 26.7°C adjusted to 6.7 | |

Magenta dyes were formed upon processing. The maximum and minimum densities to green light (D-max and D-min), the wavelength of peak absorption at a density of 1.0, and the bandwidth in nanometers at half the peak density for each example are tabulated in Table III.

**Table III**

| Example No. | D-max | D-min | Wavelength | Bandwidth |
|---|---|---|---|---|
| 1 | 2.42 | 0.08 | 549 | 92 |
| 2 | 2.28 | 0.08 | 550 | 94 |
| 3* | 2.45 | 0.08 | 548 | 90 |
| 4* | 2.31 | 0.08 | 549 | 94 |
| 5 | 2.24 | 0.08 | 548 | 94 |
| 6* | 2.08 | 0.08 | 547 | 94 |

| | | | | |
|---|---|---|---|---|
| * = comparison | | | | |

Processed strips of the coatings in Examples 1-6 were illuminated by simulated daylight at 5.4 klux for periods of 12 and 24 weeks. The green densities were monitored and the time in weeks required for 30% density loss from an initial density of 1.0 (T30) was calculated. The change in blue density in the unexposed portion of the strip after 24 weeks illumination was recorded as Printout. These data are found in Table IV.

**Table IV**

| Example No. | T30 | Printout |
|---|---|---|
| 1 | 20.0 | +.03 |
| 2 | 41.5 | +.05 |
| 3* | 16.0 | +.04 |
| 4* | 30.0 | +.05 |
| 5* | 39.4 | +.04 |
| 6* | 37.8 | +.04 |

| | | |
|---|---|---|
| * = comparison | | |

The data show that the dyes produced by couplers of our invention are more stable to light than those produced by the comparison couplers, and that their dye densities, hues and printout are at least equal to those of the comparison couplers.

## Claims

1. A photographic element comprising a support bearing at least one photographic silver halide emulsion layer and a dye-forming lH-pyrazolo [1,5-b]1,2,4 triazole coupler of the formula: 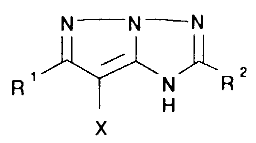 wherein
R¹ is a substituted or unsubstituted adamantyl group;
R² is a substituent group; and
X is hydrogen or a coupling-off group.

2. A photographic element as in claim 1 wherein R² is a ballast group.

3. A photographic element as in claim 1 or 2 wherein R² is represented by the formula: 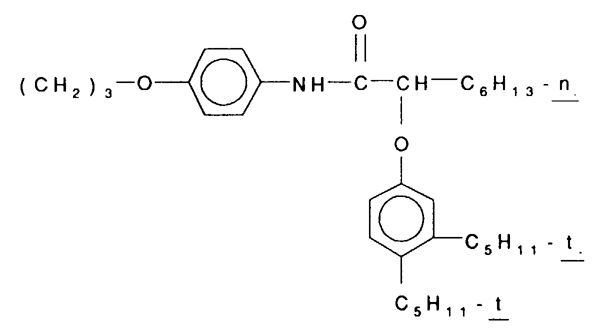 or 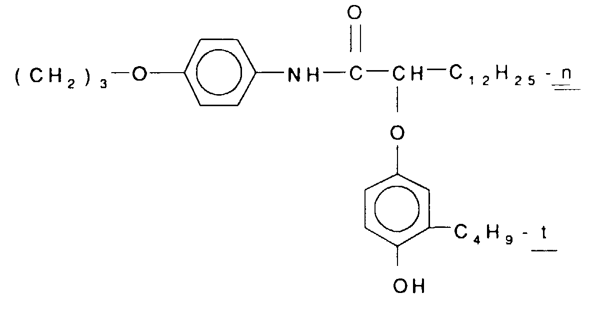 and X is Cl.

4. A process of forming a dye image in an exposed photographic element comprising a support bearing at least one photographic silver halide emulsion layer, said process comprising developing the photographic element with a color silver halide developing agent in the presence of a color coupler as defined in any of claims 1-3.

5. A 1H-pyrazolo [1,5,-b]1,2,4 triazole coupler as defined in any of claims 1-3.

6. A dye that is the coupling reaction product of an oxidized photographic color developing agent and a dye forming 1H-pyrazolo [1,5-b]1,2,4 triazole coupler as defined in any of claims 1-3.

## Patentansprüche

1. Photographisches Element mit einem Träger, auf dem sich mindestens eine photographische Silberhalogenidemulsionsschicht befindet sowie ein einen Farbstoff bildender 1H-Pyrazolo[1,5-b]1,2,4-triazolkuppler der Formel: 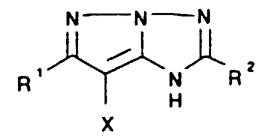 worin bedeuten:
R¹ eine substituierte oder unsubstituierte Adamantylgruppe;
R² eine Substituentengruppe; und
X Wasserstoff oder eine abkuppelnde Gruppe.

2. Photographisches Element nach Anspruch 1, worin R² eine Ballastgruppe ist.

3. Photographisches Element nach Anspruch 1 oder 2, worin R² dargestellt wird durch die Formel: 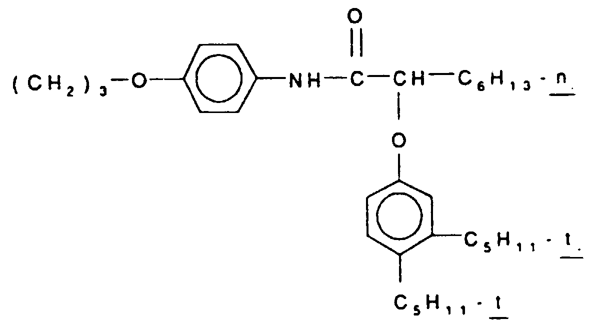 oder 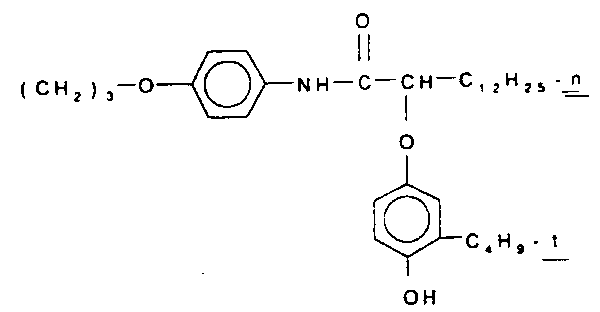 und X steht für Cl.

4. Verfahren zur Herstellung eines Farbbildes in einem exponierten photographischen Element mit einem Träger, auf dem sich mindestens eine photographische Silberhalogenidemulsionsschicht befindet, wobei das Verfahren umfaßt die Entwicklung des photographischen Elementes mit einer Silberhalogenid-Farbentwicklerverbindung in Gegenwart eines Farbkupplers nach einem der Ansprüche 1 - 3.

5. Ein 1H-Pyrazolo [1,5-b]1,2,4-triazolkuppler nach einem der Ansprüche 1 - 3.

6. Ein Farbstoff, bei dem es sich um das Kupplungs-Reaktionsprodukt einer oxidierten photographischen Farbentwicklerverbindung und einem einen Farbstoff liefernden 1H-Pyrazolo[1,5-b]1,2,4-triazolkuppler wie in einem der Ansprüche 1 - 3 definiert,handelt.

## Revendications

1. Elément photographique comprenant un support portant au moins une couche d'émulsion photographique aux halogénures d'argent et un coupleur formateur de colorant 1H-pyrazolo[1,5-b]1,2,4-triazole de formule : 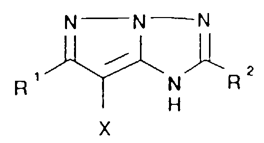 où :
R¹ est un groupe adamantyle substitué ou non ;
R² est un substituant ; et
X est l'hydrogène ou un groupe se séparant au couplage.

2. Elément photographique selon la revendication 1, où R² est un groupe ballast.

3. Elément photographique selon les revendications 1 ou 2, où R² est représenté par la formule : 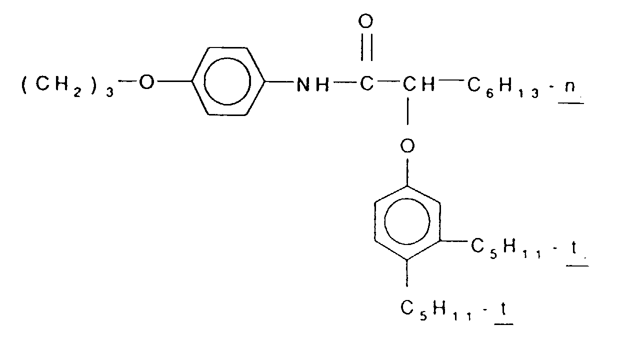 ou 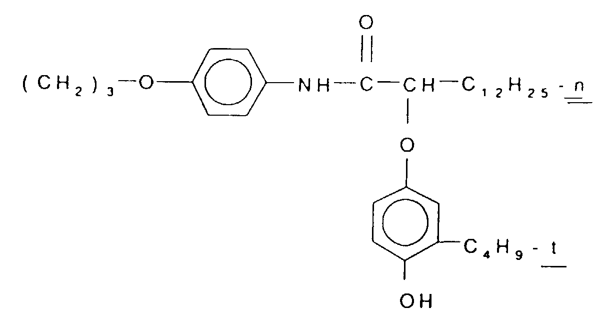 et X est Cl.

4. Procédé de formation d'une image photographique dans un élément photographique exposé comprenant un support portant au moins une couche d'émulsion photographique aux halogénures d'argent, caractérisé en ce que ledit procédé consiste à développer l'élément photographique au moyen d'un développateur chromogène des halogénures d'argent en présence d'un coupleur chromogène tel que défini dans l'une quelconque des revendications 1-3.

5. Coupleur 1H-pyrazolo[1,5-b]1,2,4-triazole tel que défini dans l'une quelconque des revendications 1-3.

6. Colorant représentant le produit de la réaction de couplage d'un développateur photographique chromogène oxydé et d'un coupleur formateur de colorant 1H-pyrazolo[1,5-b]1,2,4-triazole tel que défini dans l'une quelconque des revendications 1-3.
